# EUROPEAN PATENT APPLICATION

(11) **EP 2 067 434 A2**
(43) Date of publication of application: **10.06.2009**
(21) Application number: 08021191.5
(22) Date of filing: 05.12.2008
(51) Int. Cl.: A61B 1/005, G02B 23/24

(54) **Holding cable, and observation apparatus and endoscope apparatus including holding cable**

(30) Priority: 07.12.2007 JP 2007317370
(71) Applicant: Olympus Medical Systems Corporation, Tokyo 151-0072 (JP)
(72) Inventor: Onoda, Fumiyuki, Tokyo 151-0072 (JP); Taniguchi, Akira, Tokyo 151-0072 (JP)
(74) Representative: von Hellfeld, Axel

(57) **Abstract**

A holding cable 31 of the invention includes: a tubular body 130 including at one end thereof a fixing connector 40 and at the other end thereof a coupling portion 33e, the tubular body being configured by consecutively including a plurality of tubular members 33 having at end portions thereof inner fitting portions 35 and outer fitting portions 36, the inner and outer fitting portions being connecting portions configured as bendable joint portions 30; and a wire 34 for bringing the bendable joint portions 30 into a fixed state and fixing and holding the tubular body 130 in an arbitrary posture.

## Description

### BACKGROUND OF THE INVENTION

### 1. Field of the Invention

The present invention relates to a holding cable capable of switching between a bendable state and a bending posture fixed and held state, and an observation apparatus and an endoscope apparatus which include the holding cable.

### 2. Description of Related Art

Conventionally, endoscopes have been widely used, which enable observation of organs in a body cavity by inserting an insertion portion in the body cavity and various therapeutic treatments by introducing, as needed, a treatment instrument into the body cavity through a treatment instrument channel provided in the insertion potion.

When inserting an insertion portion of an endoscope into a body cavity, the operator usually grasps the insertion portion with his/her right hand, and grasps an operation portion with his/her left hand. The operator then operates a bending knob or various switches provided to the operation portion with his/her left hand. That is, the operator operates the bending knob or various switches provided to the operation portion while holding the operation portion by one hand during operation. Therefore, the longer an inspection time, the larger a burden placed on the operator's hand. As a result, the operator gets tired.

In view of the above-circumstances, various endoscope holding devices for holding an operation portion of an endoscope in use have been proposed in order to reduce the burden on the operator's hand during operation.

For example, Japanese Patent Application Laid-Open Publication No. 2006-247289 discloses an electric bending endoscope apparatus including an endoscope holding device. The endoscope holding device is capable of switching between two modes, that is, a usage standby state and a usage state, while the user constantly holds the electric bending endoscope, and the change between two modes of the usage standby state and the usage state can be performed very easily by a single operation.

Incidentally, the endoscope holding device disclosed in the Japanese Patent Application Laid-Open Publication No. 2006-247289 includes a supporting portion and an arm portion. Accordingly, there is a problem that an operation room becomes narrower when the endoscope holding device is arranged in the operation room. Therefore, a desire exists for a holding cable which does not spoil the space in the operation room and allowing the user to hold the operation portion of the endoscope when he or she desires.

The present invention has been achieved in view of the above-described circumstances and an object of the present invention is to provide a holding cable capable of switching between a bendable state and a state in which an arbitrarily bent posture can be fixed and held, and an observation apparatus and an endoscope apparatus including the holding cable.

### SUMMARY OF THE INVENTION

Briefly, a holding cable of the present invention includes: a tubular body including at one end thereof a fixing portion and at the other end thereof an attaching portion, the tubular body being configured by consecutively including a plurality of tubular body-forming members having at end portions thereof joint configuring portions, the joint configuring portions being connecting portions configured as bendable joint portions; and a shape-maintaining portion for bringing the bendable joint portions into a fixed state and fixing and holding the tubular body in an arbitrary posture.

In addition, an observation apparatus including a holding cable of the present invention includes an observation member for observing a subject, the observation member being provided in a fixed manner at the end portion of the tubular body configuring the holding cable according to any one of claims 1 to 7.

Furthermore, an endoscope apparatus of the present invention includes: the holding cable according to any one of claims 1 to 7; an endoscope connector provided at an end portion of the holding cable; and an endoscope fixed at the end portion of the tubular body of the holding cable.

The above and other objects, features and advantages of the invention will become more clearly understood from the following description referring to the accompanying drawings.

### BRIEF DESCRIPTION OF THE DRAWINGS

FIG. 1 is a view describing an endoscope system provided with an endoscope which has a universal cable including a holding cable.
FIG. 2 is an enlarged view of A' part in FIG. 1.
FIG. 3 is a three-side view including a front view, side view, and rear view, each of which describes a configuration of a tubular body-forming member.
FIG. 4 is a view describing a connection between the tubular body-forming members.
FIG. 5 is a view describing a bendable joint portion configured by connecting the tubular body-forming members.
FIG. 6 is a view describing working of the joint portion.
FIG. 7 is a view describing a relationship among the tubular body-forming member, a sliding piece, and a wire.
FIG. 8 is a front view describing a configuration of the sliding piece.
FIG. 9 is a view describing an exemplary configuration of a fixing connector.
FIG. 10 is a view describing a fixed state of the wire to a pulley.
FIG. 11 is a view describing working of a second pulley and a disc spring.
FIG. 12 is a view describing a fixed state of the joint portion.
FIG. 13 is a view describing a configuration in which a fixed state of the universal cable is adjusted by a switch lever provided to a coupling portion.
FIG. 14 is a view describing a configuration in which a fixed state of the universal cable is adjusted by a plurality of motors provided to the fixing connector.
FIG. 15 is a view describing a holding device.

### DETAILED DESCRIPTION OF PREFERRED EMBODIMENTS

Hereinafter, embodiments of the present invention will be described with reference to the drawings.

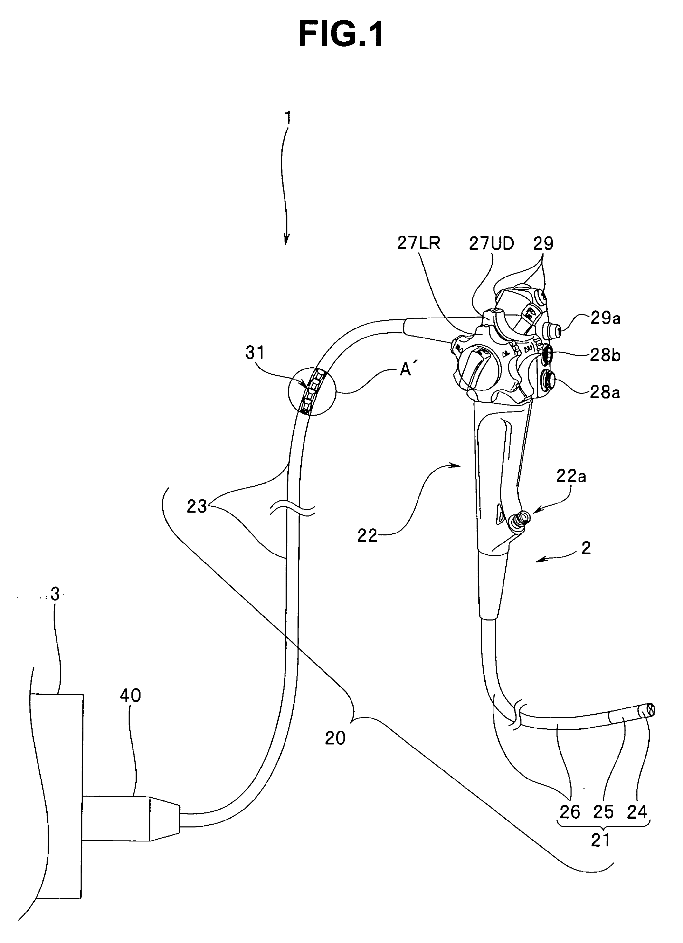

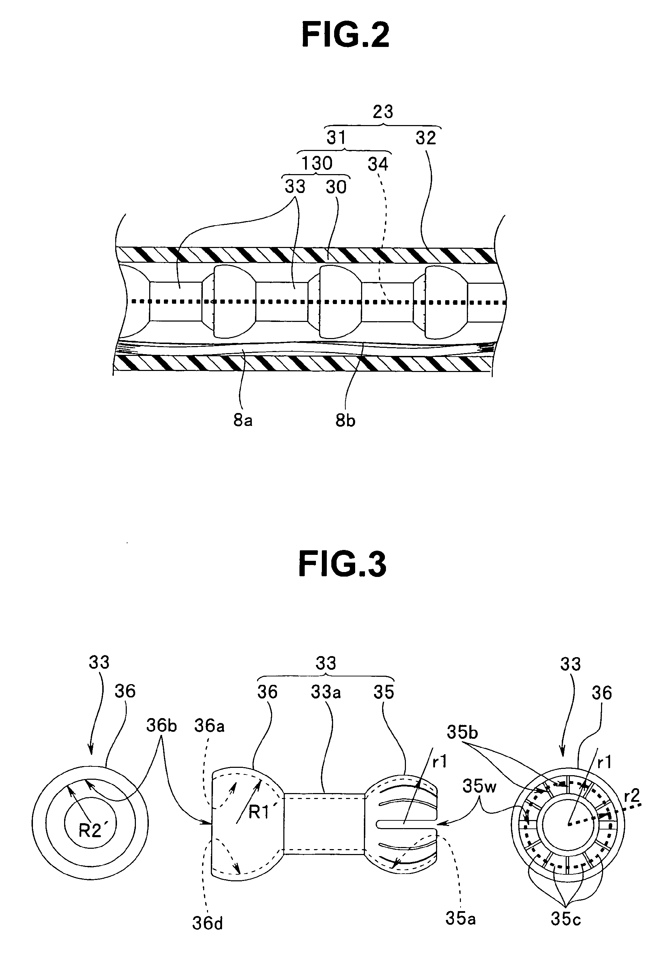

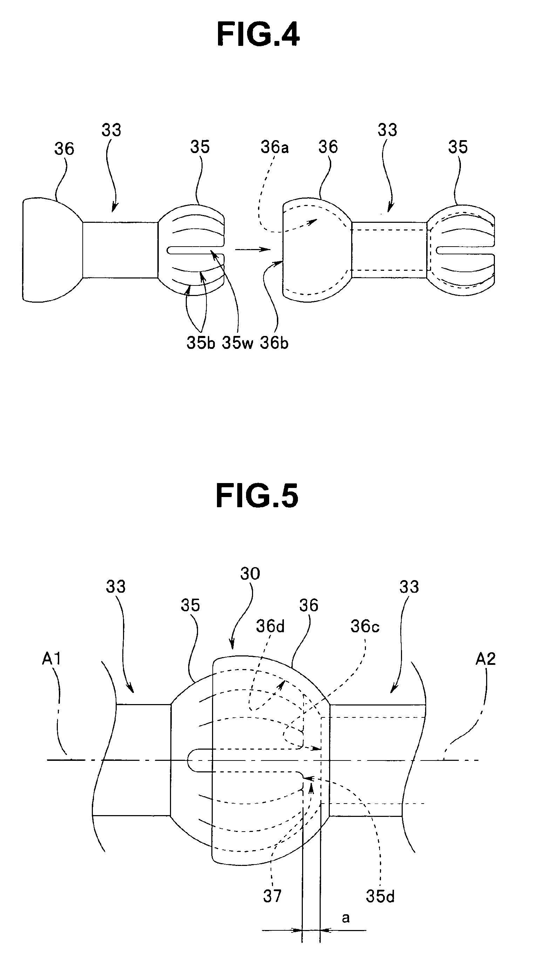

FIGS. 1 to 12 relate to the present embodiment. FIG. 1 is a view describing an endoscope system provided with an endoscope which has a universal cable including a holding cable. FIG. 2 is an enlarged view of A' part in FIG. 1. FIG. 3 is a three-side view including a front view, a side view, and rear view, each of which describes a configuration of a tubular body-forming member. FIG. 4 is a view describing a connection between the tubular body-forming members.

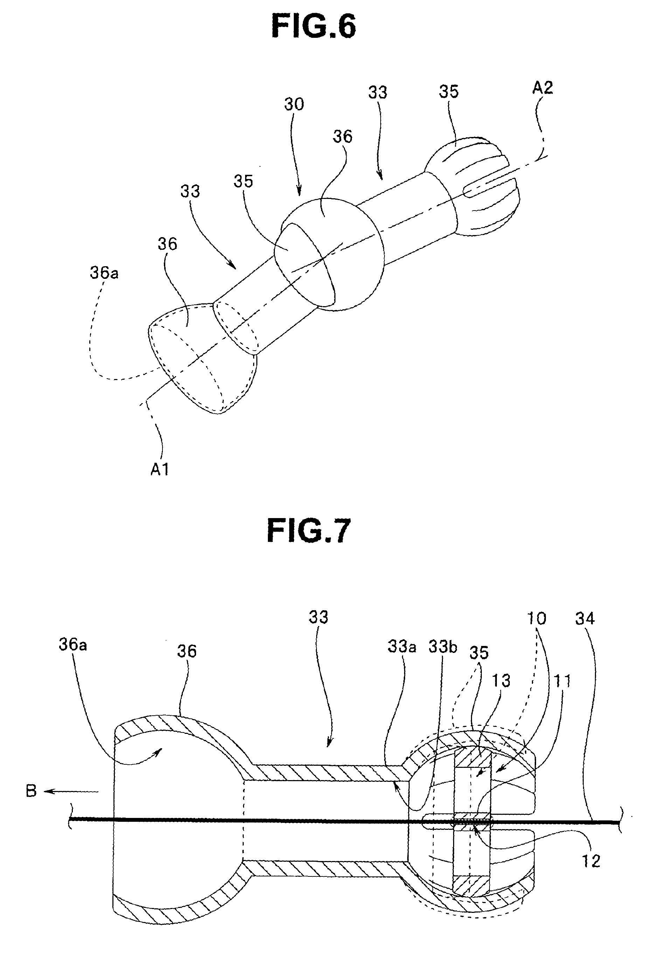

In addition, FIG. 5 is a view describing a bendable joint portion configured by connecting the tubular body-forming members. FIG. 6 is a view describing working of the joint portion. FIG. 7 is a view describing a relationship among the tubular body-forming member, a sliding piece, and a wire.

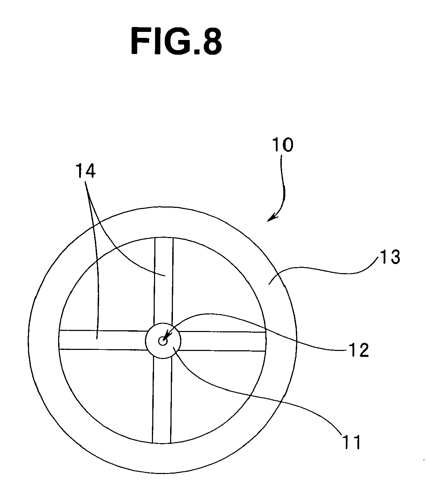

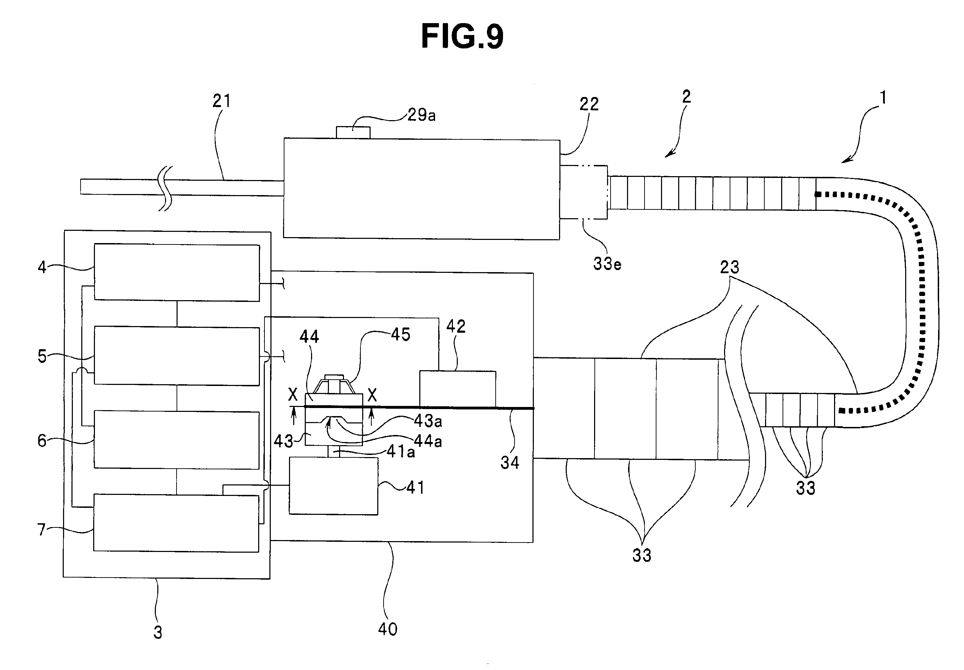

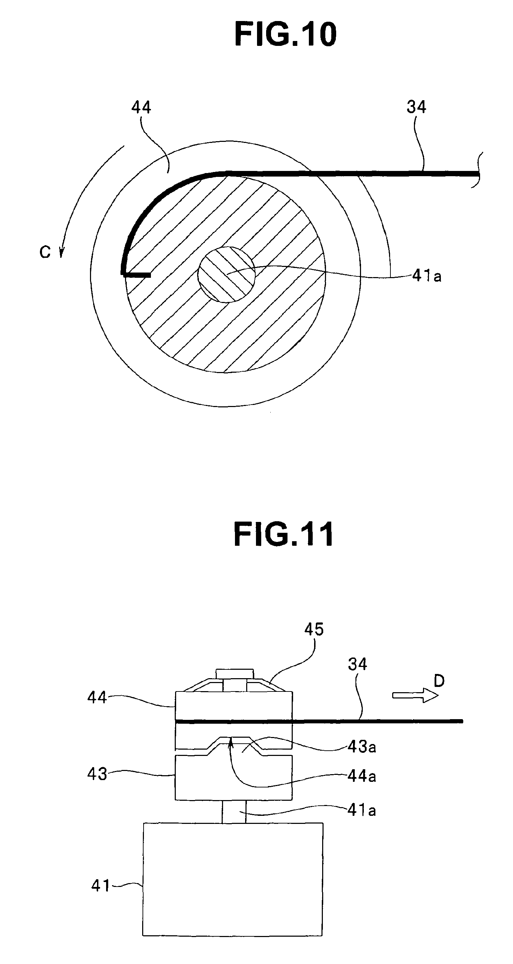

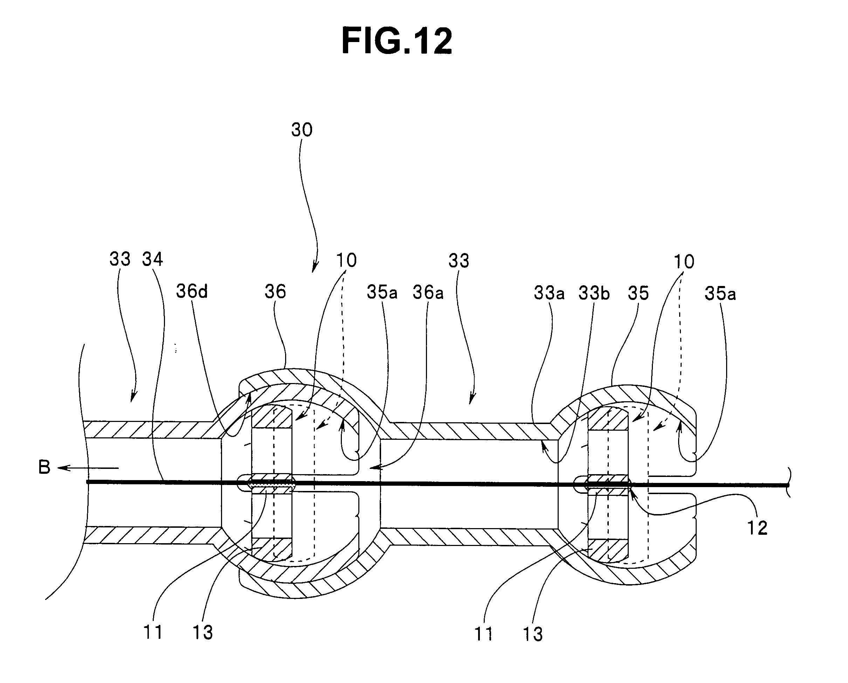

Furthermore, FIG. 8 is a front view describing a configuration of the sliding piece. FIG. 9 is a view describing an exemplary configuration of a fixing connector. FIG. 10 is a view describing a fixed state of the wire to a pulley. FIG. 11 is a view describing working of a second pulley and a disc spring. FIG. 12 is a view describing a fixed state of the joint portion.

As shown in FIG. 1, an endoscope system 1 as an endoscope apparatus is configured by including an observation apparatus 20 and, for example, an integrating apparatus 3. The observation apparatus 20 is configured by including an endoscope 2 as an observation member having an insertion portion 21 to be inserted into a body cavity and an operation portion 22 provided on a proximal end side of the insertion portion 21, and a universal cable 23 as a so-called universal cord extended from the operation portion 22.

Note that, in the present embodiment, the universal cable 23 can be switched between a flexible state and a state where an arbitrary posture is fixed and held, and is configured by including a holding cable 31 to be described later.

The insertion portion 21 is configured by including consecutively in the following order from a distal end side: a rigid distal end portion 24; a bending portion 25 bendable, for example, in up, down, left and right directions, and a flexible tube portion 26 which is long and has flexibility.

The operation portion 22 also serves as a grasping portion. The operation portion 22 is provided with: an up/down bending knob 27UD for bending the bending portion 25 in up and down directions; a left/right bending knob 27LR for bending the bending portion 25 in left and right directions; an air/water feeding button 28a; a suction button 28b; and a plurality of remote buttons 29 for instructing driving control of an image pickup unit and other units, not shown, provided to the distal end portion 24.

In addition, the operation portion 22 includes a treatment instrument insertion port 22a configuring a proximal end portion of a treatment instrument channel, not shown. Note that the reference numeral 40 represents a fixing connector, that is, a so-called endoscope connector. Note that a reference numeral 29a represents a fixing instruction switch as a hand-side operation portion to be described later.

As shown in FIGS. 1 and 2, the universal cable 23 is configured by including the holding cable 31 and an exterior covering member 32. The holding cable 31 is configured by including a bendable tubular member 130 formed by a plurality of tubular body-forming members (hereinafter abbreviated as tubular members) 33 which are consecutively provided through joint portions 30, and a pulling wire (hereinafter abbreviated as wire) 34 as a shape-maintaining portion. The exterior covering member 32 covers an outer circumference of the holding cable 31.

Note that a light guide 8a, a signal line 8b and the like inserted in the universal cable 23 are arranged, for example, in a gap formed between the exterior covering member 32 and the holding cable 31.

Specifically, as shown in the side view in FIG. 3, the tubular member 33 is configured by including a pipe-shaped main body portion 33a, an inner fitting portion 35 and an outer fitting portion 36 which have hollow and generally bulb shape and are joint configuring portions configuring the joint portion 30. The inner fitting portion 35 and the outer fitting portion 36 are provided on one end portion side and the other end portion side respectively, of the main body portion 33a.

As shown in the front view and the side view in FIG. 3, the outer fitting portion 36 has an internal space 36a whose inner diameter dimension is R1'. In the internal space 36a is disposed the inner fitting portion 35, outer diameter dimension of which is r1, of another tubular member 33 such that an outer surface of the inner fitting portion contacts the internal space.

An opening portion 36b for guiding the inner fitting portion 35 into the internal space 36a is formed at an end portion of the outer fitting portion 36. An inner diameter dimension of the opening portion 36b is R2' which is formed to be smaller than an outer diameter of the inner fitting portion 35 by a predetermined ratio. This prevents the inner fitting portion 35 from falling off from the internal space 36a of the outer fitting portion 36. Note that an end face of the opening portion 36b is formed perpendicular to a longitudinal axis of the main body portion 33a.

As shown in the rear view and the side view in FIG 3, a plurality of notch grooves 35b communicating outside with an internal space 35a are formed in the inner fitting portion 35. A pair of opposing notch grooves 35w among the plurality of notch grooves 35b is a guide groove for guiding a sliding piece to be described later (see the reference numeral 10 in FIG. 7 and the like) to the internal space 35a, and a width dimension of the pair of notch grooves is formed to be wide in consideration of a thickness of the sliding piece.

The notch grooves 35b, 35w are arranged like meridians drawn on a terrestrial globe to connect the north and south poles. The inner fitting portion 35 is configured by including a plurality of elastic pieces 35c. The inner fitting portion 35 is configured such that the outer diameter dimension thereof is changed from r1 to r2 by deforming the elastic piece 35c against an elastic force thereof.

By changing the outer diameter dimension of the inner fitting portion 35 to r2, the inner fitting portion 35 passes through the opening portion 36b and is housed in the internal space 36a of the outer fitting portion 36. The outer diameter dimension of the inner fitting portion 35 housed in the internal space 36a is changed from r2 to r1 due to the elastic force of the elastic pieces 35c. This brings the outer surface of the elastic pieces 35c, that is, the outer surface of the inner fitting portion 35 into contact with an inner surface 36d configuring the internal space 36a.

Specifically, as shown in FIG. 4, two tubular members 33 are prepared, and the inner fitting portion 35 of one of the tubular members 33 is pressed against an edge portion of the opening portion 36b of the outer fitting portion 36 of the other of the tubular members 33, to reduce the outer diameter of the inner fitting portion 35, thereby arranging the inner fitting portion 35 in the internal space 36a of the outer fitting portion 36 in a push-in manner.

Then the inner fitting portion 35 of one of the tubular members 33 is arranged in the internal space 36a of the outer fitting portion 36 of the other of the tubular members 33, as shown in FIG. 5. At this time, the inner fitting portion 35 arranged in the internal space 36a has its diameter enlarged by the elastic force of the elastic pieces 35c, thereby being disposed in the outer fitting portion 36 with the outer surface of the elastic pieces 35c contacting the inner surface 36d of the outer fitting portion 36, for example. Thus, the joint portion 30 is configured.

Note that, when an axis A1 of one of the tubular members 33 and an axis A2 of the other of the tubular members 33 are coaxial, a gap 37 having a dimension of "a", for example, is formed between a bottom portion 36c of the internal space 36a and a distal end surface 35d of the inner fitting portion 35. This configuration allows the joint portion 30 to smoothly rotate.

By connecting the two tubular members 33 through the joint portion 30, the tubular members are rotatably arranged as shown in FIG. 6, which enables the tubular body 130 to change into a state shown in FIG. 5 where the axis A1 of one of the tubular members 33 and the axis A2 of the other of the tubular members 33 are on a straight line or a state where the axis A1 and the axis A2 intersect with each other as shown in FIG. 6, for example. Then, a tubular body 130 is formed by consecutively providing a plurality of tubular members 33, and thereby the holding cable 31 is configured.

As shown in FIG. 7, the wire 34 is inserted into a through hole 12 of a wire stopper portion 11 provided to the sliding piece 10 as a shape-maintaining portion, and integrally fixed to the wire stopper portion 11 by fixing means such as adhesive, soldering, or the like.

The sliding piece 10 is a pressing member and includes the wire stopper portion 11, a contact sliding portion 13 which is an annular portion concentric with the wire stopper portion 11, and four supporting portions 14 that fixedly connect the contact sliding portion 13 and the wire stopper portion 11, for example, as shown in FIG. 8.

When the wire 34 is pulled in B direction in FIG. 7, the sliding piece 10 moves in the internal space 35a of the inner fitting portion 35 in a direction toward the main body portion 33a and enlarges the outer diameter of the inner fitting portion 35 so as to be larger than r1. Then, the inner fitting portion 35 is disposed in the outer fitting portion 36 with the outer surface of the inner fitting portion 35 in FIG. 5 changed from a state of contacting the inner surface 36d of the outer fitting portion 36 into a state of pressing the inner surface 36d of the outer fitting portion 36.

The fixing connector 40 configuring a fixing portion attachable/detachable to and from the integrating apparatus 3 is provided on a proximal end portion of the universal cable 23 having the holding cable 31 that is configured by including a plurality of tubular members 33 provided consecutively, as shown in FIG. 9.

A housing configuring the fixing connector 40 includes inside thereof a motor 41 as a state switching portion for pulling the wire 34, and a tension sensor 42 for detecting a tension of the wire 34.

The tension sensor 42 is a distortion sensor, an optical sensor, and the like, and detects whether or not the tension of the wire 34 is in a predetermined state. Detection result by the tension sensor 42 is outputted to a motor control unit 7 to be described later.

A motor shaft 41 a of the motor 41 is provided with, for example, a first pulley 43 and a second pulley 44. The first pulley 43 is integrally fixed to the motor shaft 41 a and the second pulley 44 is slidably with respect to the motor shaft 41 a.

As shown in FIG. 10, one end portion of the wire 34 is integrally fixed to the second pulley 44. The other end portion of the wire 34 is fixed to the tubular member 33 which is provided on the holding cable 31 at a position closest to the operation portion 22.

As shown in FIG. 9, on an end portion of the motor shaft 41a is provided a disc spring 45 for biasing the second pulley 44 to the first pulley 43 side with a predetermined amount of force to bring the second pulley 44 into close contact with the first pulley 43.

On a surface of the first pulley 43, which is located on the second pulley side, for example, a plurality of projection portions 43a are radially provided. On the other hand, on a surface of the second pulley 44, which is located on the first pulley side, recessed portions 44a with which the projection portions 43a are engaged are formed. On outer circumferential surfaces of the projection portions 43a and inner circumferential surfaces of the recessed portion 44a, tapered surfaces for helping a smooth engagement are formed.

According to this configuration, in the engaged state between the projection portions 43a of the first pulley 43 and the recessed portions 44a of the second pulley 44 due to the biasing force of the disc spring 45, driving the motor 41 causes the motor shaft 41 a to be rotated in an arrow C direction in FIG. 10. Thereby the first pulley 43 and the second pulley 44 are rotated in the arrow C direction to pull the wire 34.

On the other hand, in the engaged state between the projection portions 43a of the first pulley 43 and the recessed portions 44a of the second pulley 44 due to the biasing force of the disc spring 45, if a large external force is applied to the wire 34 in an arrow D direction in FIG. 11, the second pulley 44 is moved toward an end portion side of the motor shaft 41a against the biasing force of the disc spring 45.

Then the engaged state between the projection portions 43a and the recessed portions 44a is released, thereby bringing about a non-transmission state in which a rotational force of the first pulley 43 is not transmitted to the second pulley 44. By canceling the large external force in the arrow D direction, the projection portions 43a of the first pulley 43 and the recessed portions 44a of the second pulley 44 again changes into the engaged state due to the biasing force of the disc spring 45.

As shown in FIG. 9, the integrating apparatus 3 includes a light source unit 4 for supplying illumination light to an illumination optical system of the endoscope 2; a video processor unit 5 including a circuit and the like for generating a video signal from an image signal which has been photoelectrically converted and transmitted by an image pickup device, not shown, provided in the distal end portion of the endoscope 2; a power supply unit 6 for supplying electric power to the light source unit 4, the video processor unit 5, and the motor 41; and a motor control unit 7 for controlling the driving of the motor 41 based on a detection signal outputted from the tension sensor 42, for example.

In the present embodiment, when a fixing instruction switch 29a as a state switching portion provided to the operation portion 22 is operated, a fixing instruction signal is outputted from the fixing instruction switch 29a to the video processor unit 5. The video processor unit 5 which has received the fixing instruction signal transmits the fixing instruction signal to the motor control unit 7.

Then, the motor control unit 7 outputs a driving signal to the motor 41 to drive the motor 41. This causes the motor shaft 41 a to rotate in the arrow C direction in the FIG. 10, and thereby the wire 34 is pulled by the second pulley 44 which is rotated when the motor shaft 41a is rotated. As a result, the tension of the wire 34 is changed.

The change in the tension of the wire 34 is detected by the tension sensor 42, and the detection result is inputted to the motor control unit 7. Based on the inputted detection result, the motor control unit 7 controls the driving of the motor 41. The wire 34 is thus maintained so as to have a predetermined tension, and thereby the joint portions 30 become a fixed state. As a result, the holding cable 31 becomes a fixed state. That is, the universal cable 23 having the holding cable 31 is maintained in a fixed and held state.

Note that the motor is provided in the fixing connector in the present embodiment. However, the wire may be pulled by a motor provided in the integrating apparatus 3, for example.

Next, working of the present embodiment will be described.

During a procedure of ERCP (Endoscopic Retrograde Cholangiopancreatography) with the insertion portion 21 of the endoscope 2 inserted into a body cavity, for example, if the operator feels a burden of weight of the operation portion 22, he or she operates the fixing instruction switch 29a.

According to the switch operation, the instruction signal for instructing a fixed and held state of the universal cable is transmitted to the motor control unit 7 through the video processor unit 5 thereby the motor 41 is driven and the wire 34 is pulled.

Note that the fixed and held state of the universal cable means a state where the bent shape of the universal cable 23 is fixed in the posture at that time, and the operation portion 22 is held at an end portion of the fixed and held universal cable 23.

When the wire 34 is pulled in the B direction as shown in FIG. 12 by the operator's operation of the fixing instruction switch 29a, the sliding piece 10 fixed to the wire 34 is moved in the internal space 35a in a direction toward the main body portion 33a to gradually enlarge the outer diameter of the inner fitting portion 35, as described above.

This brings about a close contact state in which the outer surface of the inner fitting portion 35 presses the inner surface 36d of the internal space 36a of the outer fitting portion 36. This increases frictional resistance between the inner surface 36d of the internal space 36a provided in the outer fitting portion 36 and the outer surface of the inner fitting portion 35, thereby bringing the joint portions 30 into a fixed state.

That is, in the universal cable 23 provided with the holding cable 31, when the operator operates the fixing instruction switch 29a, the wire 34 is moved and the holding cable 31 of the universal cable 23 bent in a predetermined posture is fixed and held in the bent shape.

In this fixed and held state, when the operator releases his or her hand from the operation portion 22, the operation portion 22 is held by the fixed and held universal cable 23 in the state where the operator released his or her hand, that is, in the shape as-is shown in FIG. 1, for example.

When the operator operates the fixing instruction switch 29a again to give an instruction to release the fixed and held state of the universal cable, the driving of the motor 41 is stopped. That is, the pulled state of the wire 34 is released.

Then, the inner fitting portion 35, the diameter of which has been enlarged to be larger than r1 with the elastic force of the elastic pieces 35c, is changed into the original shape. Then the pressing force with which the outer surface of the inner fitting portion 35 presses the inner surface 36d of the outer fitting portion 36 is cancelled and the outer surface of the inner fitting portion 35 and the inner surface 36d of the outer fitting portion 36 returns to the original contact state. That is, the fixed state of the joint portions 30 is released and the universal cable 23 becomes bendable.

Thus, the bendable tubular body is configured by consecutively including the tubular members each having the outer fitting portion and the inner fitting portion thereby forming the joint portions, and the holding cable is configured by providing the wire for moving the tubular members configuring the tubular body. According to this, when the wire pulling operation is performed, the joint portions of the holding cable can be changed from the bendable state into the fixed state.

Therefore, in the endoscope having the universal cable configured by including the holding cable, when the operator operates the fixing instruction switch during the operation, thereby bringing the bendable joint portions into a fixed state and fixing and holding the universal cable in the posture of the bent shape during the operation. According to this, when the operator releases his or her hand from the operation portion, the universal cable in the fixed and held state is held in the operation state during the operation.

Note that, in the above-described embodiment, by operating the fixing instruction switch 29a, the state of the holding cable 31 configuring the universal cable 23 is assumed to be changed into two stages, that is, the bendable state and the fixed and held state.

However, the state change of the holding cable 31 is not limited to the two-stage change. The pulling state of the wire 34 may be controlled in three or more stages based on the detection result outputted from the tension sensor 42.

In this case, the fixing instruction switch 29a is configured not as a switch for instructing ON/OFF control but as a switch also serving as an adjusting portion for instructing the fixed state in stages. The fixed state includes, in addition to the above-described fixed and held state, a held state in which the position of the operation portion 22 is prevented from moving further by bringing the universal cable 23 into a state where a large force is needed to bend the universal cable. The fixed state may be set to be changeable in accordance with preference of the operator who operates the endoscope.

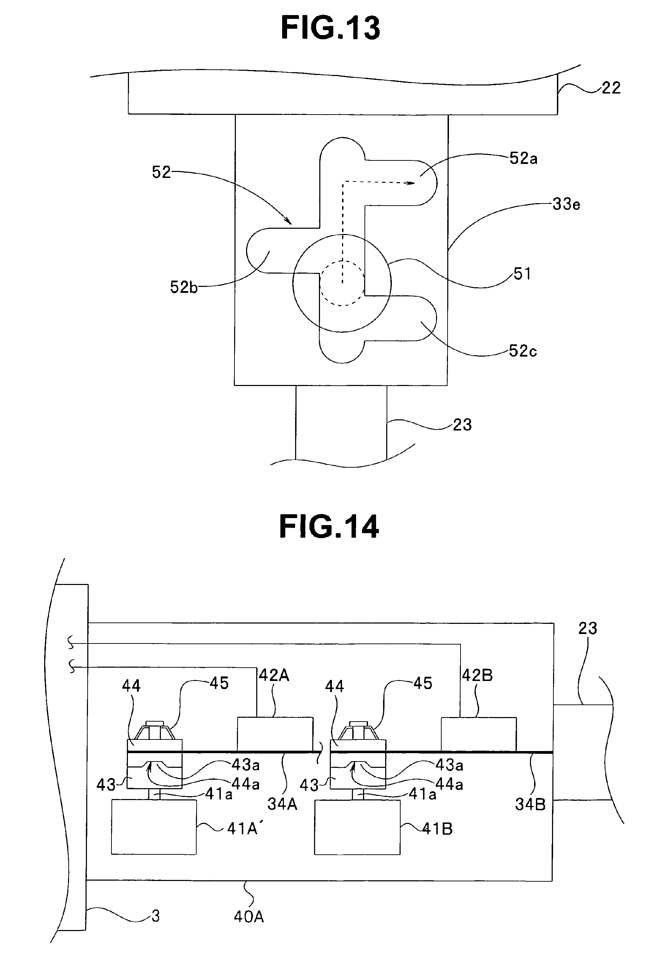

In addition, the configuration in which the state of the holding cable is switchable in a plurality of stages is not limited to one in which the states are switched based on the detection result by a sensor, and may be the configurations as shown in FIG. 13 and FIG. 14, for example.

FIG. 13 is a view describing a configuration in which a fixed state of the universal cable is adjusted by a switch lever provided to a coupling portion.

In the present embodiment, a coupling portion 33e as an attaching portion shown by a two-dot chain line in FIG. 9, for example, is provided, and a switch lever 51 as a hand-side operation portion as well as an adjusting portion shown in FIG. 13 are provided to the coupling portion 33e. The switch lever 51 is movably arranged to a switching portion 52 including switch grooves 52a, 52b and 52c, for example. By arranging the switch lever 51 in the desired switch groove 52a, for example, as shown by a dashed arrow, the fixed state of the universal cable 23 can be switched into the fixed state at a desired stage.

This configuration eliminates the need of the motor and the sensor, to configure the universal cable.

FIG. 14 is a view describing a configuration in which a fixed state of the universal cable is adjusted by a plurality of motors provided to the fixing connector.

As shown in FIG. 14, in the present embodiment, in addition to a first motor 41A', a second motor 41B is disposed in a fixing connector 40A as a state switching portion and an adjusting portion.

One end portion of a first wire 34A is fixed to a pulley 44 which is rotated by a first motor 41A', and one end portion of a second wire 34B is fixed to a pulley 44 rotated by a second motor 41B. The tensions of the wires 34A, 34B are detected by tension sensors 42A, 42B, respectively.

In the present embodiment, the first wire 34A is configured to move the tubular members 33 configuring, for example, a portion from a distal end to a midway of the holding cable, and the second wire 34B is configured to move the tubular members 33 configuring, for example, a portion from a proximal end to the midway of the holding cable 31.

According to this configuration, by pulling the first and second wires, the universal cable can be brought into the above-described fixed and held state, or by pulling the second wire, the part from a midway portion to a proximal end portion of the universal cable can be brought into a fixed and held state and the part from a distal end portion to the midway portion of the universal cable can be made bendable, and by pulling the first wire, the part from the distal end portion to the midway portion can be brought into a fixed and held state and the part from the midway portion to the proximal end portion can be made bendable. This configuration allows a moving range of the operation portion to be restricted.

Note that two motors are provided in the fixing connector. However, three or more motors may be provided.

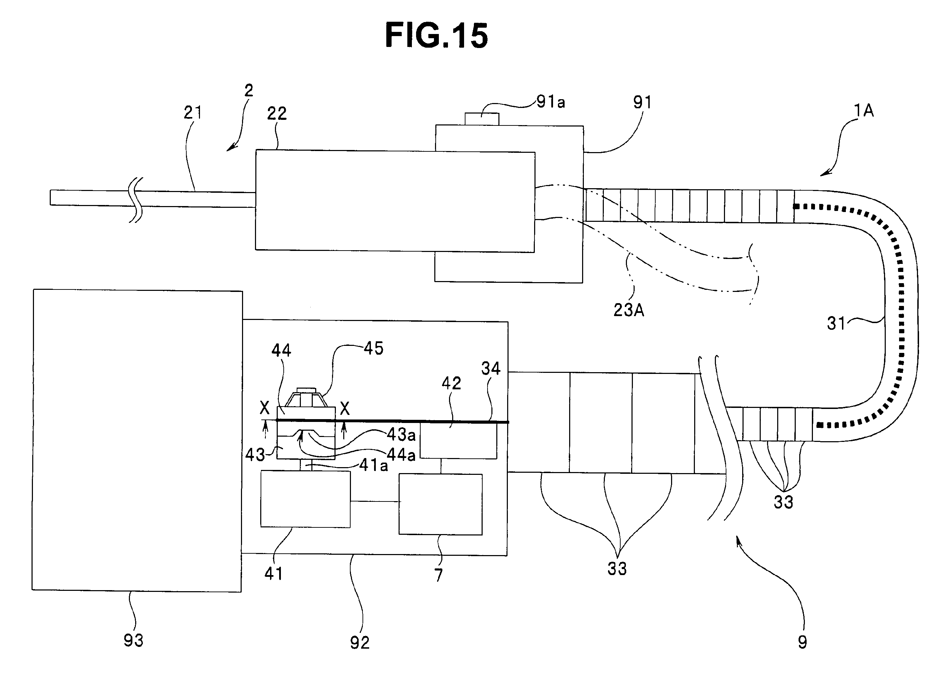

The holding cable is configured as the universal cable in the above-described embodiment. However, the holding cable may be configured as the holding device shown in FIG. 15.

FIG. 15 is a view describing the holding device.

As shown in FIG. 15, an endoscope system 1A is configured by including a universal cord 23A, the endoscope 2, and a holding device 9. The holding device 9 includes the holding cable 31, an attaching portion 91, and a fixing portion 92.

The fixing portion 92 has substantially the same configuration as that of the above-described fixing connector 40, and includes in the housing thereof the motor 41, as a state switching portion, for pulling the wire 34 and the tension sensor 42 for detecting the tension of the wire 34.

In addition, for example the motor control unit 7 is provided in the fixing portion 92. The fixing portion 92 is configured to be disposed to a fixing device 93 provided on a wall or a ceiling in an operation room, for example. Note that the fixing portion 92 is provided with a power supply cord, not shown, connected to an outlet provided on the wall of the operation room, for example.

The attaching portion 91 is a holding portion for holding and fixing in a manner sandwiching the operation portion 22 of the endoscope 2, for example. The fixing portion 91 is provided with a fixing instruction switch 91a.

Other configurations are the same as those of the above-described embodiment. The same components are attached with the same reference numerals, and descriptions thereof will be omitted.

According to this configuration, the attaching portion of the holding device is attached to the holding portion of the endoscope, thereby enabling the above-described working and effects to be obtained. Therefore, the configuration can be applied to an existing endoscope.

Note that the present invention is not limited only to the above-described embodiment, and various modifications can be made without departing from the gist of the invention.

## Claims

1. A holding cable comprising:
a tubular body including at one end thereof a fixing portion and at the other end thereof an attaching portion, the tubular body being configured by consecutively including a plurality of tubular body-forming members having at end portions thereof joint configuring portions, the joint configuring portions being connecting portions configured as bendable joint portions; and
a shape-maintaining portion for bringing the bendable joint portions into a fixed state and fixing and holding the tubular body in an arbitrary posture.

2. The holding cable according to claim 1, wherein the shape-maintaining portion includes a state switching portion for switching the joint portions between a bendable state and a fixed state.

3. The holding cable according to claim 1 or 2, further comprising
an adjusting portion for adjusting a fixed state of the joint portions which is brought into the fixed state by the shape-maintaining portion.

4. The holding cable according to claim 3, wherein the adjusting portion changes the fixed state of the joint portions in stages.

5. The holding cable according to claim 3, wherein the adjusting portion changes at least either one of the joint portions provided on one end side of the tubular body and the joint portions provided on the other end side of the tubular body into a fixed state.

6. The holding cable according to any one of claims 3 to 5, wherein the adjusting portion changes the fixed state of the joint portions included in the tubular body by a hand-side operation portion.

7. A holding cable comprising:
a tubular body configured by consecutively including a plurality of tubular body-forming members having at end portions thereof joint configuring portions, the joint configuring portions being connecting portions between the tubular body-forming members, the connecting portions being configured as rotatable joint portions;
a wire inserted into the tubular body and provided in a fixed manner to the plurality of tubular body-forming members; and
a pressing member for pressing the connecting portions and changing a fixed state of the joint portions by pulling the wire.

8. An observation apparatus including a holding cable, comprising
an observation member for observing a subject, the observation member being provided in a fixed manner at the end portion of the tubular body configuring the holding cable according to any one of claims 1 to 7.

9. The observation apparatus including the holding cable according to claim 8, wherein the observation member is an endoscope.

10. An endoscope apparatus comprising:
the holding cable according to any one of claims 1 to 7;
an endoscope connector provided at an end portion of the holding cable; and
an endoscope fixed at the end portion of the tubular body of the holding cable.
